# EUROPÄISCHE PATENTANMELDUNG

(11) **EP 1 231 200 A1**
(43) Veröffentlichungstag der Anmeldung: **14.08.2002**
(21) Anmeldenummer: 02001393.4
(22) Anmeldetag: 19.01.2002
(51) Int. Cl.: C07C 51/09, C07C 67/343

(54) **Verfahren zur Herstellung von cis-Hexadec-6-en-säure**

(30) Priorität: 07.02.2001 DE 10105799; 22.02.2001 DE 10108603
(71) Anmelder: BASF AKTIENGESELLSCHAFT, 67056 Ludwigshafen (DE)
(72) Erfinder: Klein, Daniela, Dr., 68161 Mannheim (DE); Ernst, Hansgeorg, Dr., 67346 Speyer (DE); Koppenhöfer, Jürgen, 67435 Neustadt (DE)

(57) **Zusammenfassung**

Die Erfindung betrifft ein Verfahren zur Herstellung von cis-Hexadec-6-ensäure der Formel I, 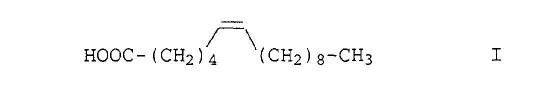 dadurch gekennzeichnet, daß man
a₁) ein Triphenylphosphoniumsalz der allgemeinen Formel II,

   R¹OOC-(CH₂)₅-P(R²)₃⁺ X⁻ II

   mit Decanal der Formel III

   H₃C-(CH₂)₈-CH=O III

   oder
a₂) ein Triphenylphosphoniumsalz der allgemeinen Formel IV,

   H₃C-(CH₂)₉-P(R²)₃⁺ X⁻ IV

   mit einem Aldehyd der Formel V

   R¹OOC-(CH₂)₄-CH=O V

   in einer Wittig-Reaktion umsetzt und
b) den nach Verfahrensschritt a₁) oder a₂) gebildeten Ester der allgemeinen Formel VI, 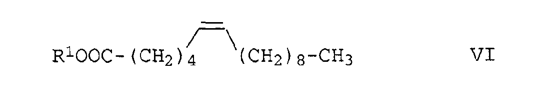 verseift, wobei die Substituenten R¹, R² und X⁻ die in der Beschreibung genannte Bedeutung haben.

## Beschreibung

Die Erfindung betrifft ein Verfahren zur Herstellung von cis-Hexadec-6-ensäure.

cis-Hexadec-6-ensäure (sapienic acid) ist im menschlichen Hauttalg die am häufigsten vorkommende ungesättigte Fettsäure. Aufgrund ihrer antimikrobiellen Wirkung ist cis-Hexadec-6-ensäure für kosmetische und dermatologische Anwendungen ein gefragter Wirkstoff.

Zur Herstellung von cis-Hexadec-6-ensäure bzw. deren Ester sind sowohl mikrobiologische als auch chemische Verfahren bekannt.

So beschreibt WO 96/13591 die stereoselektive Wasserstoffabspaltung von Palmitylestern mittels Mikroorganismen.

Ein chemischer Syntheseweg zur Gewinnung von cis-Hexadec-6-ensäure ist beschrieben in WO 98/16104. Die vielstufige Synthese liefert jedoch nur geringe Ausbeuten und verwendet darüberhinaus sicherheitstechnisch bedenkliche Reagenzien wie z.B. flüssigen Ammoniak und Kaliumcyanid.

Es war die Aufgabe der vorliegenden Erfindung, ein neues Verfahren zur Herstellung von cis-Hexadec-6-ensäure bereitzustellen, das mit einer hohen Z/E-Selektivität verläuft und in sicherheitstechnischer Hinsicht die Nachteile der bisher bekannten Verfahren nicht aufweist.

Diese Aufgabe wurde gelöst durch ein Verfahren zur Herstellung von cis-Hexadec-6-ensäure der Formel I, 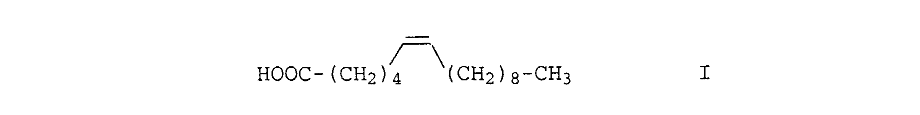 dadurch gekennzeichnet, daß man
a₁) ein Triphenylphosphoniumsalz der allgemeinen Formel II,

   R¹OOC-(CH₂)₅-P(R²)₃⁺ X⁻ II

   in der die Substituenten unabhängig voneinander folgende Bedeutung haben:
   - R¹: C₁-C₁₂-Alkyl, Aryl;
   - R²: Aryl und
   - X⁻: ein Anionenäquivalent einer anorganischen oder organischen Säure
   mit Decanal der Formel III

   H₃C-(CH₂)₈-CH=O III

   in einer Wittig-Reaktion umsetzt oder
a₂) ein Triphenylphosphoniumsalz der allgemeinen Formel IV,

   H₃C-(CH₂)₉-P(R²)₃⁺ X⁻ IV

   in der die Substituenten R² und X⁻ unabhängig voneinander die oben genannte Bedeutung haben:
   mit einem Aldehyd der Formel V,

   R¹OOC-(CH₂)₄-CH=O V

   in der R¹ die oben genannte Bedeutung hat,
   in einer Wittig-Reaktion umsetzt
   und
b) den nach Verfahrensschritt a₁) oder a₂) gebildeten Ester der allgemeinen Formel VI, 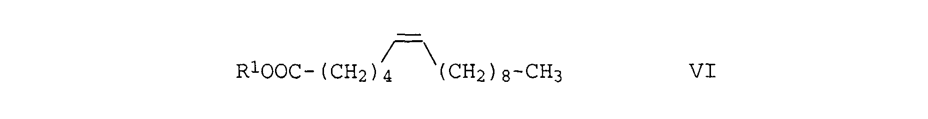 in der R¹ die oben genannte Bedeutung hat, verseift.

Als Alkylreste für R¹ seien verzweigte oder unverzweigte C₁-C₁₂-Alkylketten wie Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl genannt. Bevorzugte Alkylreste sind C₁-C₄-Alkylgruppen, besonders bevorzugt Methyl, Ethyl, n-Propyl und 1-Methylethyl, ganz besonders bevorzugt Methyl und Ethyl.

Unter Aryl für R¹ sind aromatische Ringe oder Ringsysteme mit 6 bis 18 Kohlenstoffatomen im Ringsystem zu verstehen, beispielsweise Phenyl oder Naphthyl, die ggf. mit einem oder mehreren Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein können. Bevorzugt sind Phenyl, Methoxyphenyl und Naphthyl.

Der Begriff Aryl für R² bezeichnet übliche, in Phosphinen und Phosphoniumsalzen vorkommende Arylreste, wie Phenyl, Toluol, Naphthyl, ggf. jeweils substituiert, bevorzugt Phenyl.

Der Rest X⁻ steht für ein Anionenäquivalent einer anorganischen oder organischen Säure, bevorzugt einer starken anorganischen oder organischen Säure.

Der Ausdruck starke Säure umfaßt Halogenwasserstoffsäuren (insbesondere Salzsäure und Bromwasserstoffsäure), Schwefelsäure, Phosphorsäure, Sulfonsäuren und andere anorganische oder organische Säuren mit vergleichbarem Dissoziationsgrad. Als starke organische Säuren sind in diesem Zusammenhang auch C₁-C₆-Alkansäuren zu verstehen.

Besonders bevorzugt sind Anionen solcher Säure, ausgewählt aus der Gruppe, bestehend aus Salzsäure, Bromwasserstoffsäure, Schwefelsäure, Phosphorsäure, Ameisensäure, Essigsäure und Sulfonsäure zu nennen. Ganz besonders bevorzugt Cl⁻, Br⁻, CₙH₂ₙ₊₁-SO₃⁻ (mit n = 1-4), Ph-SO₃⁻, p-Tol-SO₃⁻ oder CF₃-SO₃⁻.

Gegenstand der Erfindung ist insbesondere ein Verfahren zur Herstellung von cis-Hexadec-6-ensäure, dadurch gekennzeichnet, daß man im Verfahrensschritt a) ein Triphenylphosphoniumsalz der Formel II mit Decanal der Formel III umsetzt.

Eine weitere bevorzugte Ausführungsform des Verfahrens ist dadurch gekennzeichnet, daß man ein Triphenylphosphoniumsalz der Formel IIa verwendet,

R¹OOC-(CH₂)₄-P(Ph)₃⁺ X⁻ IIa

in der die Substituenten unabhängig voneinander folgende Bedeutung haben:
- R¹: C₁-C₄-Alkyl, insbesondere Methyl, Ethyl, n-Propyl und 1-Methylethyl;
- Ph: Phenyl;
- X⁻: Anionenäquivalent einer starken anorganischen oder organischen Säure, insbesondere Cl⁻, Br⁻, CₙH₂ₙ₊₁-SO₃⁻ mit n = 1-4, Ph-SO₃⁻, p-Tol-SO₃⁻ oder CF₃-SO₃⁻.

Die Umsetzung der Phosphoniumsalze II oder IV zu den cis-Hexadec-6-ensäureestern der Formel VI kann unter den für Wittig-Reaktionen üblichen Bedingungen erfolgen.

Die Reaktion im Schritt a) erfolgt in der Regel bei Temperaturen zwischen -30°C und +50°C, bevorzugt zwischen -10 und +30°C, besonders bevorzugt zwischen +10°C und +25°C.

Man kann dabei entweder beide Ausgangsverbindungen (Phosphoniumsalz und Aldehyd) in dem Lösungsmittel vorlegen und dazu die Base geben oder aber eine Lösung des Phosphoniumsalzes vorlegen, die Base zufügen und erst danach eine Lösung des Aldehyds addieren.

Als Base können alle für Wittig-Kondensationen üblichen Basen verwendet werden, z.B. Alkalimetallhydroxide wie Natriumhydroxid, Kaliumhydroxid oder Lithiumhydroxid; Alkalimetallhydride wie Natriumhydrid oder Kaliumhydrid.

Als Basen kommen außerdem Lithiumorganyle wie z.B. n-Butyllithium, tert. Butyllithium, Phenyllithium oder Alkalimetallamide wie Lithium-, Kalium- oder Natriumamid, Lithium-diisopropylamid aber auch Alkalimetallhexamethyldisilazide in Frage. Als bevorzugte Base für die erfindungsgemäße Wittig Reaktion werden Natrium- oder Kaliumhexamethyldisilazid sowie Kalium- oder Natriumamid eingesetzt.

Die Menge an eingesetzter Base liegt in der Regel im Bereich von 0,8 bis 5 Mol, bevorzugt 1 bis 3 Mol pro Mol des eingesetzten Phosphoniumsalzes II oder IV.

Als Lösungsmittel für den Verfahrensschritt a) kommen u.a. aromatische Kohlenwasserstoffe wie Toluol, Xylol oder Benzol, cyclische oder offenkettige Ether wie Diethylether, Diisopropylether, Methyl-tert.butylether, 1,4-Dioxan oder THF sowie DMF oder DMSO in Frage. Bevorzugte Lösungsmittel sind Toluol, THF und/oder DMSO.

Nach vollständiger Umsetzung wird das Reaktionsgemisch hydrolysiert und der gebildete Ester extraktiv aus der wäßrigen Lösung entfernt.

Als Extraktionsmittel verwendet man mit Vorteil Hexan, Heptan oder Essigsäureethylester. Es können aber auch alle anderen mit Wasser nicht mischbaren organischen Lösungsmittel, wie Ether, aliphatische Kohlenwasserstoffe, halogenierte und aromatische Kohlenwasserstoffe zur Extraktion verwendet werden.

Die Lösungsmittel, insbesondere das DMF oder DMSO bleiben bei dieser Extraktion weitgehend in der wäßrigen Phase und halten auch das bei der Wittig-Reaktion gebildete Triphenylphosphinoxid weitgehend in der wäßrigen Phase zurück.

Das erfindungsgemäße Verfahren ist dadurch gekennzeichnet, daß die Wittig-Reaktion im Verfahrensschritt a) mit einer Z/E Selektivität größer 90/10, bevorzugt mit einer Z/E Selektivität zwischen 92/8 und 99/1, besonders bevorzugt zwischen 94/6 und 97/3 erfolgt.

Die Verseifung im Verfahrensschritt b) erfolgt in der Regel derart, daß man den cis-Hexadec-6-ensäureester in einem C₁-C₆-Alkohol, bevorzugt Ethanol, n-Propanol, iso-Propanol oder Butanol, besonders bevorzugt in Ethanol vorlegt und mit einer Base, beispielsweise eine wäßrige oder wäßrig-alkoholische Lösung eines Alkalimetall- oder Erdalkalimetallhydroxids, bevorzugt eine wäßrig-ethanolische Natronlauge oder Kalilauge versetzt.

Die Reaktionstemperaturen in der Stufe b) liegen im Bereich zwischen 0°C und der Siedetemperatur des Lösungsmittels, bevorzugt zwischen 10°C und 100°C, besonders bevorzugt im Bereich zwischen 30°C und 80°C.

Die zur Verseifung des Esters verwendete Base wird mindestens in stöchiometrischer Menge, bezogen auf das Edukt VI eingesetzt. Die Base kann aber auch in mehrfach molarem Überschuß, bevorzugt in 2 bis 10-fachem molaren Überschuß, besonders bevorzugt in 3 bis 8-fachem molaren Überschuß, bezogen auf das Edukt VI, eingesetzt werden.

Anhand der folgenden Beispiele soll das erfindungsgemäße Verfahren näher erläutert werden.

### Beispiel 1

### Umsetzung von 6-Bromhexansäureethylester mit Triphenylphosphin

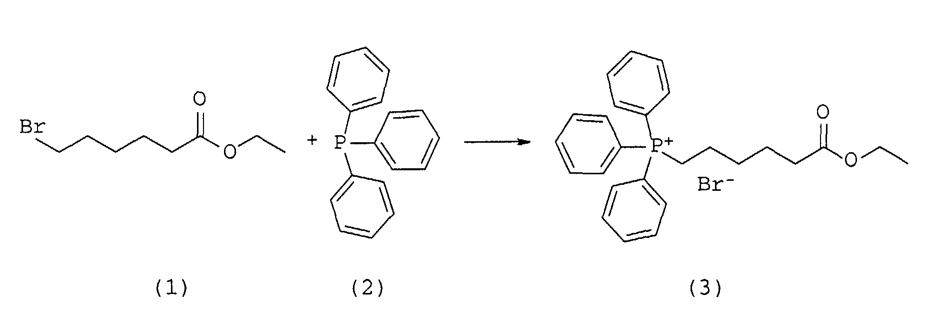

In einem 1 l Zweihalskolben wurden 112,7 g (0,5 Mol) 6-Bromhexansäureethylester (1) (99 %ig) zusammen mit 132,5 g (0,5 Mol) Triphenylphosphin (2) (99 %ig) 6 Stunden in 600 ml Xylol unter Rückfluß gekocht. Nach den 6 Stunden wurde abgekühlt und das Xylol vom zähen Wertprodukt abdekantiert. Der Rückstand wurde zweimal mit je 600 mL Toluol aufgekocht, abgekühlt und das Toluol jeweils abdekantiert. Das restliche Lösungsmittel wurde am Rotationsverdampfer abdestilliert. Es blieben 181,1 g eines glasartigen, leicht gelblich gefärbten Rohproduktes zurück. Das Produkt entsprach laut ¹H- und ¹³C-NMR-Analytik der Verbindung (3). Die Ausbeute betrug 76% der Theorie.

### Beispiel 2

### Umsetzung des Phosphoniumsalzes mit Decanal zu cis-Hexadec-6-ensäureethylester

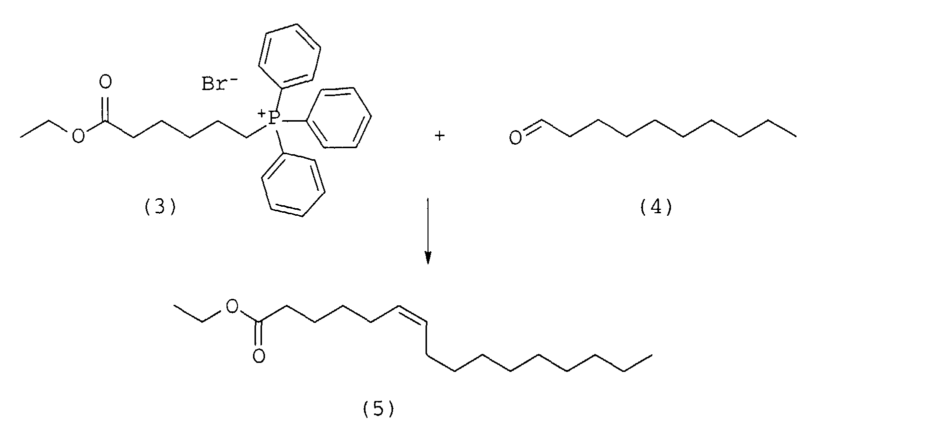

In einem 2 l Vierhalskolben mit Blattrührer, Thermometer und N₂-Blasenzähler wurden 134,8 g (0,25 Mol) des nach Beispiel 1 erhaltenen Phosphoniumsalzes (3) zusammen mit 37,3 g (0,227 Mol) Decanal (4) in 680 ml DMSO vorgelegt. Unter Rühren wurden bei 20-25°C eine Lösung aus 43,8 g (0,227 Mol) NaHMDS 95%ig und 226 ml DMSO innerhalb von 15 Minuten zugetropft. Es wurde 24 Stunden bei RT nachgerührt. Anschließend wurde der Ansatz auf 1100 ml wässrige 1N HCl und 2000 ml n-Hexan gegeben. Die Wasserphase wurde nochmals mit 1000 ml n-Hexan extrahiert. Die vereinigten org. Phasen wurden mit jeweils 500 ml 5%iger wäßriger NaHCO₃-Lsg. und Wasser gewaschen, dann mit Na₂SO₄ getrocknet und am Rotationsverdampfer eingeengt. Der Rückstand wurde in 1000 ml n-Heptan gelöst und 5 x mit je 100 mL Wasser/Methanol (1:1 v/v) gewaschen. Die Heptanphase wurde mit Na₂SO₄ getrocknet und anschließend das Heptan am Rotationsverdampfer abdestilliert. Der Rückstand wurde auf eine Glasfilternutsche mit Kieselgel gegeben und mit Cyclohexan/Essigester (2/1) eluiert. Das Lösungsmittel wurde am Rotationsverdampfer abdestilliert. Es bleiben 38,9 g Rückstand zurück. Das Produkt entsprach laut ¹H- und ¹³C-NMR-Analytik der Verbindung (5), 95% Z-Isomer, 5 % E-Isomer. Die Ausbeute betrug 35% der Theorie. Die Aufreinigung erfolgte destillativ.

### Beispiel 3

### Verseifung des cis-Hexadec-6-ensäureethylester zur cis-Hexadec-6-ensäure

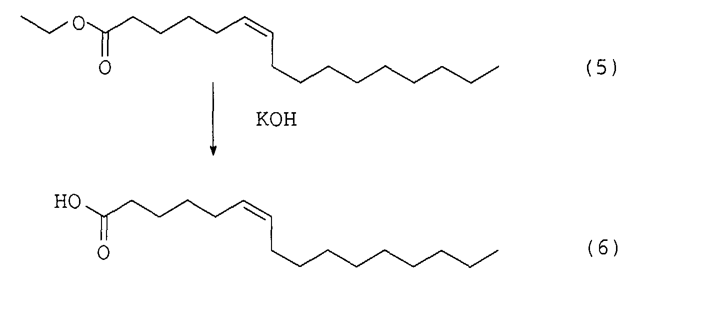

In einem 100 ml Dreihalskolben mit Magnetrührer, Rückflußkühler und N₂-Blasenzähler wurden 3,0 g des rohen cis-Hexadec-6-ensäureethylesters (5) (6,16 mMol) zusammen mit 37,8 ml (37,8 mMol) Kalilauge in Ethanol c = 1 Mol/l und 18 ml Wasser vorgelegt. Nach 2 Stunden Erhitzen auf Rückfluß wurde am Rotationsverdampfer das Lösungsmittel weitgehend abgezogen. Der Rückstand wurde mit 30 ml Eiswasser versetzt und mit 30 ml Essigester extrahiert. Man säuerte die org. Phase mit 4 ml konc. Salzsäure an. Die org. Phase wurde mit jeweils 5 ml Wasser und ges. Kochsalzlösung gewaschen, mit Na₂SO₄ getrocknet und das Lösungsmittel am Rotationsverdampfer abdestilliert. Die erhaltenen 2,59 g gelbliche Substanz entsprachen laut ¹H- und ¹³C-NMR-Analytik der cis-Hexadec-6-ensäure (6), 95% Z-Isomeres, 5% E-Isomeres. Die Ausbeute betrug 95% der Theorie. Die weitere Aufreinigung der cis-Hexadec-6-ensäure erfolgte durch Säulenchromatographie. Man erhielt ein Produkt mit einer chemischen Reinheit von größer 95%.

## Patentansprüche

1. Verfahren zur Herstellung von cis-Hexadec-6-ensäure der Formel I, 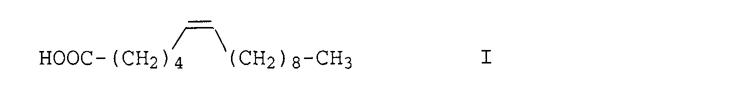 **dadurch gekennzeichnet, daß** man
a₁) ein Triphenylphosphoniumsalz der allgemeinen Formel II,
R¹OOC-(CH₂)₅-P(R²)₃⁺ X⁻ II
in der die Substituenten unabhängig voneinander folgende Bedeutung haben:
R¹ C₁-C₁₂-Alkyl, Aryl;
R² Aryl und
X⁻ ein Anionenäquivalent einer anorganischen oder organischen Säure
mit Decanal der Formel III
H₃C-(CH₂)₈-CH=O III
in einer Wittig-Reaktion umsetzt oder
a₂) ein Triphenylphosphoniumsalz der allgemeinen Formel IV,
H₃C-(CH₂)₉-P(R²)₃⁺ X⁻ IV
in der die Substituenten R² und X⁻ unabhängig voneinander die oben genannte Bedeutung haben:
mit einem Aldehyd der Formel V,
R¹OOC-(CH₂)₄-CH=O V
in der R¹ die oben genannte Bedeutung hat,
in einer Wittig-Reaktion umsetzt
und
b) den nach Verfahrensschritt a₁) oder a₂) gebildeten Ester der allgemeinen Formel VI, 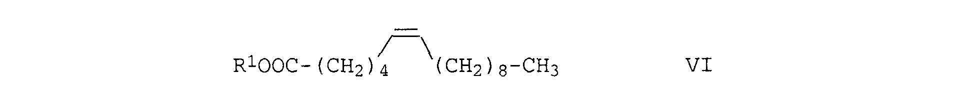 in der R¹ die oben genannte Bedeutung hat, verseift.

2. Verfahren nach Anspruch 1, **dadurch gekennzeichnet, daß** man im Verfahrensschritt a) ein Triphenylphosphoniumsalz der Formel II mit Decanal der Formel III umsetzt.

3. Verfahren nach Anspruch 2, **dadurch gekennzeichnet, daß** man ein Triphenylphosphoniumsalz der Formel IIa verwendet,
R¹OOC-(CH₂)₄-P(Ph)₃⁺ X⁻ IIa
in der die Substituenten unabhängig voneinander folgende Bedeutung haben:
R¹ C₁-C₄-Alkyl;
Ph Phenyl;
X⁻ Anionenäquivalent einer starken anorganischen oder organischen Säure.

4. Verfahren nach einem der Ansprüche 1 bis 3, **dadurch gekennzeichnet, daß** X⁻ das Anionenäquivalent einer Säure, ausgewählt aus der Gruppe, bestehend aus Halogenwasserstoffsäure, Schwefelsäure, Phosphorsäure, Ameisensäure, Essigsäure und Sulfonsäure ist.

5. Verfahren nach Anspruch 4, **dadurch gekennzeichnet, daß** X⁻ für Cl⁻, Br⁻, CₙH₂ₙ₊₁-SO₃⁻ mit n = 1-4, Ph-SO₃⁻, p-Tol-SO₃⁻ oder CF₃-SO₃⁻ steht.

6. Verfahren nach einem der Ansprüche 1 bis 5, **dadurch gekennzeichnet, daß** die Wittig-Reaktion im Verfahrensschritt a) mit einer Z/E Selektivität größer 90/10 erfolgt.
